# FASCICULE DE BREVET EUROPEEN

(11) **EP 0 640 612 B1**
(45) Date de publication et mention de la délivrance du brevet: **22.04.1998**
(21) Numéro de dépôt: 94401926.4
(22) Date de dépôt: 31.08.1994
(51) Int. Cl.: C07F 9/60, A61K 31/675, C07F 9/576

(54) **Nouveaux dérivés de 2-(1H)-quinoléinone, leur procédé de préparation et les compositions pharmaceutiques qui les contiennent**
Neue Derivate des 2-(1H)-Chinolinones, Verfahren zu ihrer Herstellung und sie enthaltende Arzneimittel
Novel derivatives from 2-(1H)-quinolinone, process for their preparation and pharmaceutical compositions containing them

(30) Priorité: 31.08.1993 FR 9310379
(43) Date de publication de la demande: 01.03.1995
(73) Titulaire: ADIR ET COMPAGNIE, 92415 Courbevoie Cédex (FR)
(72) Inventeur: Cordi, Alex, F-92150 Suresnes (FR); Desos, Patrice, F-92400 Courbevoie (FR); Lepagnol, Jean, F-28210 Chaudon (FR)

(56) Documents cités:
- EP-A- 0 542 609
- US-A- 4 124 588

## Description

La présente invention concerne de nouveaux dérivés de 2-(1H)-quinoléinone, leur procédé de préparation et les compositions pharmaceutiques qui les contiennent.

Quelques dérivés de 2-(1H)-quinoléinone ont été décrits dans la littérature. C'est le cas, par exemple, des composés décrits par C. ALABASTER et coll. (J. Med. Chem., 31, 2048-2056, 1988) qui sont des stimulants cardiaques ou de ceux décrits par F. BAHR et coll. (Pharmazie, 36, H.10, 1981).

Les composés décrits dans la présente invention, outre le fait qu'ils soient nouveaux, présentent des propriétés pharmacologiques particulièrement intéressantes : ce sont de puissants inhibiteurs des phénomènes liés à l'hyperactivation des aminoacides excitateurs.

L'acide L-glutamique et l'acide L-aspartique ont la capacité d'activer les neurones du système nerveux central et de nombreux travaux ont démontré que ces aminoacides excitateurs (AAE) répondent aux critères définissant un neurotransmetteur. C'est pourquoi la modulation des événements neuronaux liés à ces AAE apparait être une cible intéressante pour le traitement des maladies neurologiques.

En effet, il a été prouvé que la libération exagérée des AAE et l'hyperstimulation de leurs récepteurs seraient une des causes de la dégénérescence neuronale que l'on observe dans l'épilepsie, la démence sénile ou les accidents vasculaires cérébraux. Actuellement, le nombre de maladies neurodégénératives dans lesquelles les AAE sont étroitement impliqués ne cesse de grandir (chorée de Huntington, schizophrénie, sclérose amyotrophique latérale) (Mc GEER E.G. et al., Nature, 263, 517-519, 1976; SIMON R. et al., Science, 226, 850-852, 1984).

De plus, s'il est certain que l'hyperactivation de la neurotransmission aux AAE exerce des effets neurotoxiques, l'activation normale de celle-ci facilite les performances mnésiques et cognitives (LYNCH G. & BAUDRY M., Science, 224, 1057-1063, 1984 ; ROTHMAN S.M. & OLNEY J.W., Trends in Neuro Sci., 10, 299-302, 1987). Du point de vue pharmacologique et thérapeutique, il convient donc de ne s'opposer qu'aux stimulations pathologiques tout en respectant le niveau d'activation physiologique.

Les récepteurs aux AAE à localisation post- et présynaptique ont été classés en 4 groupes en fonction de l'affinité et des effets électrophysiologiques et/ou neurochimiques de ligands spécifiques :
. récepteur NMDA (N-méthyl-D-aspartate) associé à un canal ionique perméable aux cations mono- et divalents (dont le calcium) mais qui est bloqué par le magnésium. L'accumulation du calcium dans la cellule serait une des causes de la mort neuronale. L'ouverture du canal NMDA est régulée par plusieurs sites associés au récepteur et en particulier est favorisée par la glycine dont l'effet est strychnine-insensible. Ce site glycine constitue l'une des cibles importantes pour moduler l'activation du récepteur NMDA.
. récepteur AMPA (acide α-amino-3-hydroxy-5-méthyl-4-isoxazole-propionique) associé à un canal ionique perméable aux cations monovalents dont le sodium. L'activation de ce canal entrainerait une dépolarisation membranaire.
. récepteur kainate dont les caractéristiques ioniques sont proches du récepteur AMPA mais qui s'en diffère par les niveaux de conductance et de désensibilisation. Toutefois, de nombreuses études tendent à prouver que le récepteur AMPA et le récepteur kainate ont d'étroites analogies structurales et fonctionnelles et constituent une même famille réceptorielle (KEINANEN K. et al., Science, 249, 556-560, 1990).
. récepteur ACPD (acide-trans-1-aminocyclopentane-1,3-dicarboxylique) appelé récepteur métabotropique car non couplé à un canal ionique.

L'activation des récepteurs ionotropiques par les AAE ouvre les canaux ioniques et notamment permet l'entrée de sodium qui dépolarise la cellule. Cette première phase qui implique le récepteur AMPA, conduit alors à l'hyperactivation du récepteur NMDA et à l'accumulation massive de calcium (BLAKE J.F. et al., Neurosci. Letters, 89, 182-186, 1988 ; BASHIR Z.I. et al., Nature, 349, 156-158, 1991).

Les composés de la présente invention ou les produits de leur hydrolyse métabolique (pro-drogues) visent donc de manière originale à s'opposer aux effets excitateurs et toxiques des AAE en bloquant l'activation initiale du récepteur à l'AMPA/kainate.

Les composés de la présente invention sont donc utiles en tant qu'inhibiteurs des phénomènes pathologiques notamment neurotoxiques liés à l'hyperactivation des voies de neurotransmission aux aminoacides excitateurs. Ils se singularisent vis-à-vis des produits décrits par la demande de brevet européen EP 542609 en ce qu'ils sont particulièrement solubles dans l'eau, dépourvus de toute coloration et qu'ils disposent d'une bonne biodisponibilité cérébrale après administration systémique.

Ils sont donc des agents thérapeutiques potentiels pour le traitement des maladies neurologiques et psychiques impliquant ces aminoacides : maladies dégénératives aigües ou chroniques, telles que l'accident vasculaire cérébral, le traumatisme cérébral ou spinal, l'épilepsie, les maladies neurodégénératives chroniques telles que la maladie d'Alzheimer, la schizophrénie, la sclérose amyotrophique latérale ou la chorée de Huntington.

Plus spécifiquement, la présente invention concerne les composés de formule (I) : 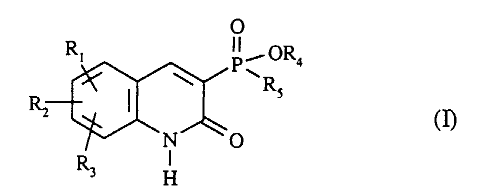 dans laquelle :
- R₁, R₂, R₃,: identiques ou différents, représentent un atome d'hydrogène, d'halogène, un groupement alkyle (C₁-C₆) linéaire ou ramifié (substitué ou non par un ou plusieurs atomes d'halogène), un groupement nitro, cyano ou aminosulfonyl,
ou bien, lorsque deux d'entre eux sont situés sur des carbones adjacents, forment avec les atomes de carbone auxquels ils sont attachés, un cycle cycloalkyle (C₃-C₇) ou un cycle benzénique (substitué ou non par un ou plusieurs atomes d'halogène, groupements alkyle (C₁-C₆) linéaire ou ramifié, alkoxy (C₁-C₆) linéaire ou ramifié ou trihalogénométhyle),
- R₄: représente un atome d'hydrogène, un groupement alkyle (C₁-C₆) linéaire ou ramifié, phényle (substitué ou non par un ou plusieurs atomes d'halogène, groupements alkyle (C₁-C₆) linéaire ou ramifié, alkoxy (C₁-C₆) linéaire ou ramifié ou trihalogénométhyle) ou un groupement 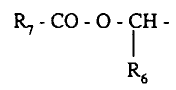 dans lequel R₆ ou R₇, identiques ou différents, représentent un atome d'hydrogène ou un groupement alkyle (C₁-C₆) linéaire ou ramifié (substitué ou non par un groupement cycloalkyle (C₃-C₇) ou phényle),
- R₅: représente un atome d'hydrogène, un groupement hydroxy, alkoxy (C₁-C₆) linéaire ou ramifié, phénoxy, mercapto, alkylthio (C₁-C₆) linéaire ou ramifié, alkyle (C₁-C₆) linéaire ou ramifié (substitué ou non par un groupement cycloalkyle (C₃-C₇)), phényle (substitué ou non par un ou plusieurs atomes d'halogène, groupements alkyle (C₁-C₆) linéaire ou ramifié, alkoxy (C₁-C₆) linéaire ou ramifié ou trihalogénométhyle), amino (substitué ou non par un ou deux groupements alkyle (C₁-C₆) linéaire ou ramifié) ou un groupement 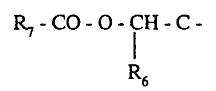 dans lequel R₆ ou R₇ sont tels que définis précédemment,
leurs isomères ainsi que leurs sels d'addition à un acide ou à une base pharmaceutiquement acceptable.

Parmi les bases pharmaceutiquement acceptables, on peut citer à titre non limitatif l'hydroxyde de sodium, l'hydroxyde de potassium, la tertbutylamine, la diéthylamine, l'éthylènediamine...

L'invention s'étend également au procédé de préparation des composés de formule (I) caractérisé en ce que l'on utilise comme produit de départ un composé de formule (II) : 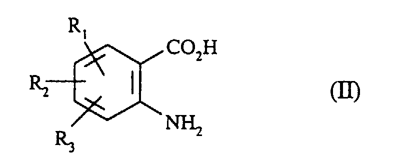 dans laquelle R₁, R₂ et R₃ ont la même signification que dans la formule (I),
que l'on réduit selon la nature des substituants R₁, R₂ et R₃,
soit en milieu aprotique en présence d'hydrure de lithium aluminium, d'hydrure d'aluminium, de diborane ou des complexes de borane,
soit en milieu protique acide lorsque l'on utilise le cyanoborohydrure de sodium, pour conduire à un alcool de formule (III) : 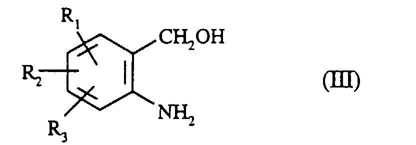 dans laquelle R₁, R₂ et R₃ ont la même signification que dans la formule (I),
que l'on oxyde à l'aide d'un oxyde métallique dans un solvant inerte, d'un hydrohalogénate de métal alcalin dans un solvant protique ou d'un chlorure d'acide dans le diméthylsulfoxyde, pour conduire à l'aldéhyde de formule (IV) : 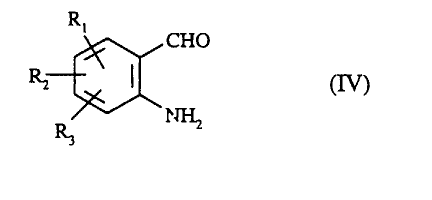 dans laquelle R₁, R₂ et R₃ ont la même signification que dans la formule (I),
qui est alors condensé, avec un malonate d'alkyle de formule (V), en milieu protique, en présence d'un alcoolate de métal alcalin, d'une amine tertiaire ou d'un hydroxyde de métal alcalin : 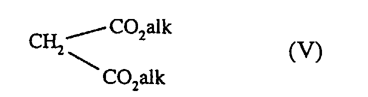 dans laquelle alk représente un groupement alkyle (C₁-C₆) linéaire ou ramifié,
pour conduire à un ester de formule (VI) : 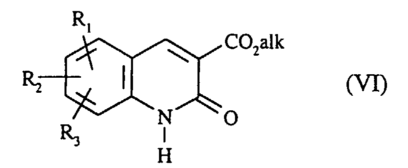 dans laquelle R₁, R₂, R₃ et alk ont la même signification que précédemment,
que l'on transforme, en acide correspondant de formule (VII) : 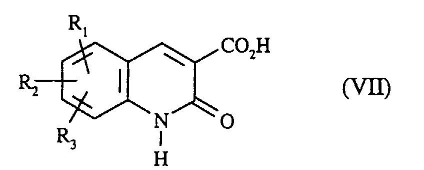 dans laquelle R₁, R₂ et R₃ ont la même signification que dans la formule (I),
que l'on met en réaction en présence de brome dans de la pyridine, pour conduire au composé de formule (VIII) : 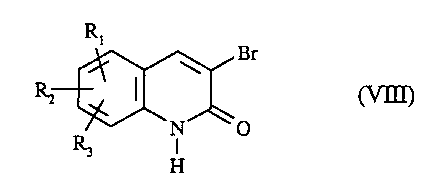 dans laquelle R₁, R₂ et R₃ ont la même signification que dans la formule (I),
dont on protège la fonction lactame par action d'oxychlorure de phosphore suivie de celle d'un alcoolate de métal alcalin en milieu alcoolique, pour conduire au composé de formule (IX) : 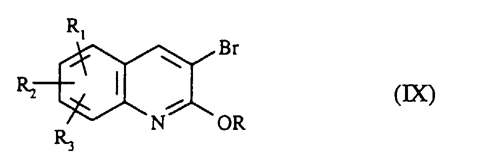 dans laquelle R₁, R₂, R₃ ont la même signification que dans la formule (I) et R représente un groupement alkyle (C₁-C₆) linéaire ou ramifié,
que l'on fait réagir avec un phosphite de dialkyle ou un diaryle en présence de triéthylamine et de tétrakis(triphényl-phosphine)palladium comme catalyseur, en milieu anhydre, selon la technique décrite par T. HIRAO (Synthesis, 56-57, 1981), pour conduire au composé de formule (X) : 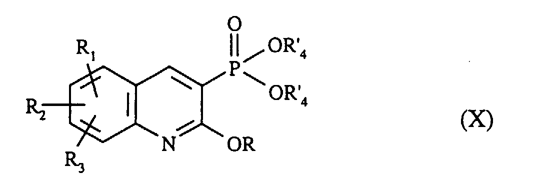 dans laquelle R₁, R₂, R₃ et R ont la même signification que précédemment et R'₄ représente un groupement alkyle (C₁-C₆) linéaire ou ramifié ou un groupement aryle,
qui est ensuite :
- soit: totalement déprotégé en présence de bromure de triméthylsilane en milieu acétonitrile puis traité en milieu acide,
pour conduire au composé de formule (I/a), cas particulier des composés de formule (I) : 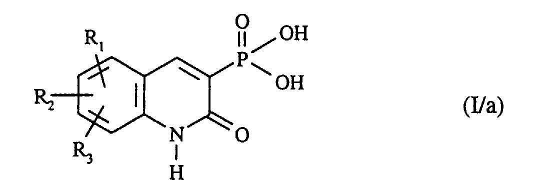 dans laquelle R₁, R₂ et R₃ ont la même signification que dans la formule (I), que l'on met en réaction si on le souhaite, en milieu basique avec un dérivé halogéné de formule R''₄-Cl dans laquelle R''₄ représente un groupement alkyle (C₁-C₆) linéaire ou ramifié, phényle (substitué ou non par un ou plusieurs atomes d'halogène, groupements alkyle (C₁-C₆) linéaire ou ramifié, alkoxy (C₁-C₆) linéaire ou ramifié ou trihalogénométhyle)
ou un groupement 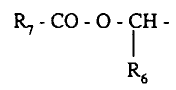 dans lequel R₆ et R₇ ont la même signification que dans la formule (I),
pour conduire au composé de formule (I/b), cas particulier des composés de formule (I), 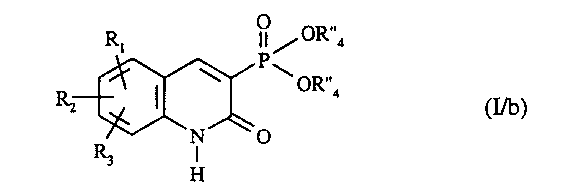 dans laquelle R₁, R₂, R₃ et R''₄ ont la même signification que précédemment,
- soit: partiellement déprotégé par traitement en milieu basique, pour conduire au composé de formule (XI) : 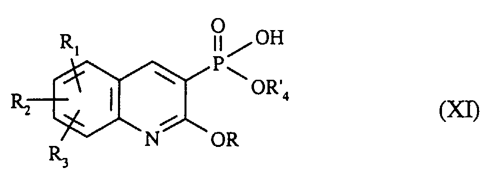 dans laquelle R₁, R₂, R₃, R et R'₄ ont la même signification que précédemment,
composé de formule (XI) :
qui subit, selon la nature des composés de formule (I) que l'on souhaite obtenir :
* ou bien : un traitement en milieu acide, pour conduire au composé de formule (I/c), cas particulier des composés de formule (I) : 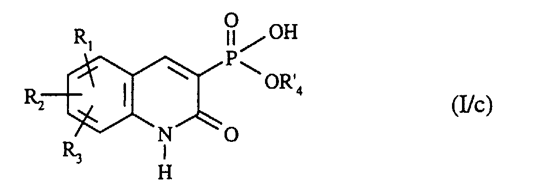 dans laquelle R₁, R₂, R₃ et R'₄ ont la même signification que précédemment,
* ou bien : l'action d'un alcool de formule (XII), selon le procédé décrit par D.A. CAMPBELL (J. Org. Chem., 57, 6331-6335, 1992) :

   R'₅ - OH (XII)

   dans laquelle R'₅ représente un groupement alkyle (C₁-C₆) linéaire ou ramifié ou phényle, pour conduire au composé de formule (XIII) : 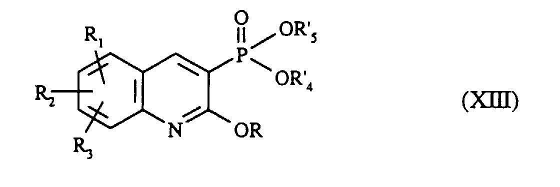 que l'on déprotège sélectivement par action du bromure de triméthylsilane en milieu acétonitrile puis traitement par de l'acide chlorhydrique, pour conduire au composé de formule (I/d), cas particulier des composés de formule (I) : 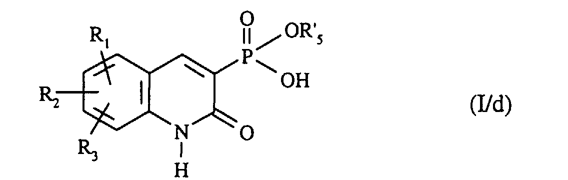 dans laquelle R₁, R₂, R₃ et R'₅ ont la même signification que précédemment,
* ou bien encore : l'action du chlorure d'oxalyle selon la méthode décrite par R. S. RODGERS (Tet. Lett., 33, 7473, 1992), pour conduire au composé de formule(XIV) : 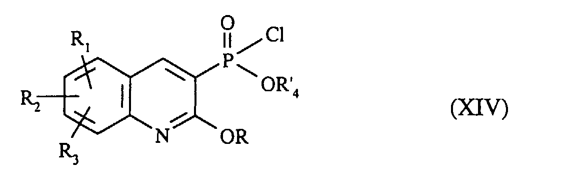 dans laquelle R₁, R₂, R₃, R et R'₄ ont la même signification que précédemment,
composé de formule (XIV) que l'on traite ensuite :
- soit avec un organomagnésien de formule (XV), en milieu anhydre :

   R''₅ MgX (XV)

   dans laquelle X représente un atome d'halogène et R''₅ représente un groupement alkyle (C₁-C₆) linéaire ou ramifié (substitué ou non par un groupement cycloalkyle (C₃-C₇)) ou phényle (substitué ou non par un ou plusieurs atomes d'halogène, groupements alkyle (C₁-C₆) linéaire ou ramifié, alkoxy (C₁-C₆) linéaire ou ramifié ou trihalogénométhyle), pour conduire au composé de formule (XVI) : 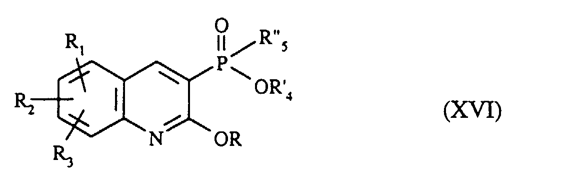 dans laquelle R₁, R₂, R₃, R, R'₄ et R''₅ ont la même signification que précédemment,
   que l'on déprotège par action du bromure de triméthylsilane en milieu acétonitrile puis traitement par de l'acide chlorhydrique,
   pour conduire au composé de formule (I/e), cas particulier des composés de formule (I) : 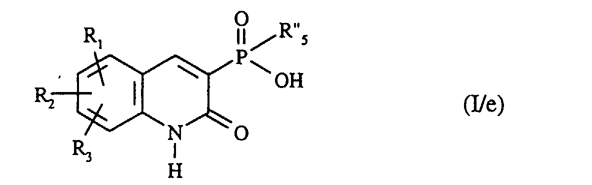 dans laquelle R₁, R₂, R₃ et R''₅ ont la même signification que précédemment,
- soit que l'on met en réaction avec un hydrure, H₂S, alkSH (dans lequel alk signifie un groupement alkyle (C₁-C₆) linéaire ou ramifié) ou de l'ammoniac, pour conduire au composé de formule (XVII) : 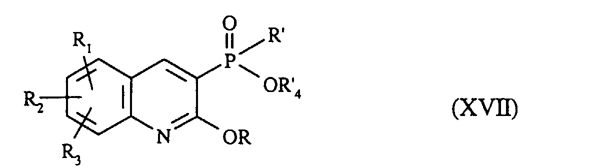 dans laquelle R₁, R₂, R₃, R et R'₄ ont la même signification que précédemment et R' représente HS-, alkS- ou H₂N-, ou un atome d'hydrogène,
   que l'on déprotège par action du bromure de triméthylsilane en milieu acétonitrile puis traitement par de l'acide chlorhydrique,
   pour conduire au composé de formule (I/f), cas particulier des composés de formule (I) : 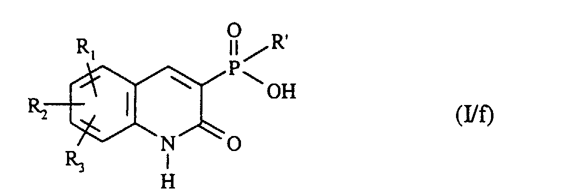 dans laquelle R₁, R₂, R₃ et R' ont la même signification que précédemment,
composé de formule (I/a), (I/b), (I/c), (I/d), (I/e) ou (I/f)
- qui, lorsque R₁ et/ou R₂ et/ou R₃ représentent un atome d'hydrogène, peut subir des substitutions électrophiles, selon des techniques classiques de substitution des noyaux aromatiques conduisant à un composé de formule (I) mono, di ou trisubstitué sur le noyau phényl de la quinoléinone,
- qui, lorsque R₁ et/ou R₂ et/ou R₃ représentent un groupement nitro, peut subir une hydrogénation pour conduire au dérivé amino correspondant, qui, lui-même, peut être, si on le souhaite, transformé en dérivé cyano correspondant,
- qui peut être, le cas échéant, purifié selon une technique classique de purification,
- dont on sépare, le cas échéant, les isomères selon une technique classique de séparation,
- que l'on transforme, si on le souhaite, en ses sels d'addition à un acide ou à une base pharmaceutiquement acceptable.

Les composés de formule (I) possèdent des propriétés pharmacologiques très intéressantes.

Le double mécanisme d'action des composés de la présente invention a pu être étudié grâce aux techniques de liaison membranaire et d'électrophysiologie.

### 1/ Binding membranaire

Il est réalisé à partir d'un homogénat de cortex de Rat. Le culot membranaire est classiquement préparé dans un tampon saccharose par centrifugations différentielles puis congelé jusqu'à utilisation. Après décongélation, 200 µl d'homogénat de membranes sont repris dans un tampon Tris HCl (30 mM), CaCl₂ (2,5 mM), pH 7,4 et incubés à 4°C pendant 30 minutes en présence de 25 µl d'une solution standard de ³H-AMPA ou de ³H-Kainate et de 25 µl d'une solution standard du produit à tester. La liaison non-spécifique est déterminée en présence de 10 µM de quisqualate (binding AMPA) ou de 10 µM d'acide kainique (binding kainate). Les membranes sont ensuite isolées par filtration. Après séchage des filtres, la radioactivité est mesurée par scintillation.

### 2/ Courants induits par les AAE dans l'oocyte de Xenopus

Des oocytes de Xenopus sont injectés de 50 ng d'ARNm poly (A⁺) isolés de cortex cérébral de Rat et incubés 2 à 3 jours à 18°C pour en permettre l'expression protéique. Les courants entrants induits par les AAE sont mesurés dans un milieu de composition : NaCl (82,5 mM), KCl (2,5 mM), CaCl₂ (1 mM), MgCl₂ (1 mM), NaH₂PO₄ (1 mM), HEPES (5 mM), pH 7,4 par la méthode du Voltage-clamp à 2 électrodes (potentiel = - 60 mV). Pour la mesure des courants induits par le NMDA et la glycine, MgCl₂ est absent du milieu et le CaCl₂ est amené à la concentration de 2 mM. Les agonistes AAE induisant les courants sont utilisés aux concentrations suivantes : kainate ; 100 µM ; AMPA : 30 µM ; glycine/NMDA : 3/30 µM. Le produit étudié est appliqué 30 secondes avant et durant l'application de l'agoniste.
Les composés de la présente invention ont été étudiés en comparaison aux plus récents composés décrits pour leur interaction avec le récepteur AMPA : ce sont des dérivés de quinoxaline-2,3-dione et plus particulièrement le 6-cyano-7-nitroquinoxaline-2,3-dione (CNQX) et le 6-nitro-7-sulfamoyl-benzo[f]quinoxaline-2,3-dione (NBQX).

Les études de binding ont montré que les dérivés de l'invention se lient sur le récepteur AMPA avec une intensité très intéressante puisque le Ki est inférieur à 10⁻⁶M.
CNQX Ki = 0,07 µM
Exemple 2 Ki = 0,9 µM

De la même manière, ils sont capables d'inhiber l'activation fonctionnelle du courant induite par le kainate et l'AMPA dans l'oocyte de Xenopus exprimant les récepteurs du glutamate.
Courant Kainate Exemple 2 IC50 = 2 µM

Cette inhibition est robuste et constante puisque la même IC50 (2µM) est retrouvée vis-à-vis des courants excitateurs neurotoxiques induits dans l'hippocampe par stimulation des voies glutamatergiques (collatérales de Schaeffer).
Bien que l'activité des composés de l'invention vis-à-vis des récepteurs AMPA/Kainate soit plus faible que celles des quinoxalinediones, leur intérêt thérapeutique, tient dans le fait qu'ils exercent une inhibition sélective du récepteur AMPA sans toucher au récepteur NMDA, ce qui les rend dépourvus de tous les effets secondaires décrits pour les antagonistes NMDA (effets psychotomimétiques, amnésiants et neurotoxiques).

De plus, les composés de l'invention présentent une activité in vivo unique et exceptionnelle pour les produits de cette classe. En effet, dans le test des convulsions audiogènes chez la souris DBA/₂ pratiqué selon la méthodologie décrite par CROUCHER et coll. (Science, 216, 899, 1982), le produit de l'exemple 2 s'oppose à ces convulsions glutamate-dépendantes et cet effet protecteur est non seulement observé après administration IP (ID50 = 18,7 mg/kg) mais aussi après administration orale et l'index de biodisponibilité (ID50 IP / ID50 PO) est voisin de 0,3 alors que les quinoxalinediones telles que le CNQX ou le NBQX sont dépourvues de toute activité par voie orale.

La présente invention a également pour objet les compositions pharmaceutiques renfermant comme principe actif au moins un composé de formule (I), seul ou en combinaison avec un ou plusieurs excipients ou véhicules inertes non toxiques.

Parmi les compositions pharmaceutiques selon l'invention on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 1 et 1000 mg pour un traitement en 1 à 3 prises par 24 heures.

Les exemples suivants illustrent l'invention et ne la limitent en aucune façon. Les produits de départ utilisés sont des produits connus ou préparés selon des modes opératoires connus.

### Exemple 1 : Acide 5,7-dichloro-2(1H)-quinoléinone-3-phosphonique

### Stade A : 3-Bromo-5,7-dichloro-2(1H)-quinoléinone

A une suspension contenant 25,4 mmoles d'acide 5,7-dichloro-2(1H)-quinoléinone-3-carboxylique dans 65 ml de pyridine à 0°C, on additionne goutte à goutte 50,8 mmoles de brome. Après 10 minutes d'agitation à température ambiante, l'ensemble est porté une heure à 90°C. Après refroidissement, 300 ml d'acide chlorhydrique 1N sont ajoutés. Le produit attendu est obtenu par filtration du précipité puis lavage de celui-ci à l'acide chlorhydrique 1N, à l'eau et séchage.
Point de fusion : > 300°C

### Stade B : 3-Bromo-2,5,7-trichloroquinoléine

9,2 mmoles du composé décrit au stade précédent sont agitées 12 heures à reflux dans 30 ml d'oxychlorure de phosphore. Après évaporation de l'excès d'oxychlorure de phosphore, le mélange est refroidi dans un bain de glace puis hydrolysé par addition d'eau glacée. Le précipité formé est filtré, rincé à l'eau et séché et conduit au produit attendu.
Point de fusion : 176-181°C

### Stade C : 3-Bromo-5,7-dichloro-2-méthoxyquinoléine

Une suspension contenant 9 mmoles du composé décrit au stade précédent et 20 ml d'une solution méthanolique 5,2M de méthanolate de sodium est portée à reflux 12 heures. Après refroidissement, le produit attendu est obtenu après filtration du précipité.
Point de fusion : 135-138°C

### Stade D : Diéthylester de l'acide 5,7-dichloro-2-méthoxyquinol-3-yl phosphonique

A 6,5 mmoles du composé décrit au stade précédent en suspension dans 3 ml de tétrahydrofurane anhydre sont ajoutées 13 mmoles de triéthylamine, 13 mmoles de diéthylphosphite et 0,65 mmoles de tétrakis (triphénylphosphine) palladium. L'ensemble est porté à reflux, sous atmosphère d'azote, pendant 12 heures. La solution est alors diluée dans 200 ml d'acétate d'éthyle. La phase organique est lavée par de l'acide chlorhydrique 1N puis par une solution saturée de chlorure de sodium. Après séchage et évaporation, le produit attendu est obtenu après purification du résidu par chromatographie sur colonne de silice en utilisant comme éluant un mélange cyclohexane/acétate d'éthyle (1/1) puis acétate d'éthyle.
Point de fusion : 66-68°C

| Microanalyse élémentaire : | | | | |
|---|---|---|---|---|
| | C % | H % | N % | Cl % |
| calculé | 46,18 | 4,43 | 3,85 | 19,47 |
| trouvé | 46,29 | 4,33 | 3,95 | 19,15 |

### Stade E : Acide 5,7-dichloro-2(1H)-quinoléinone-3-phosphonique

A une solution contenant 2 mmoles du composé décrit au stade précédent dans 10 ml d'acétonitrile anhydre, sont ajoutés 2 ml de bromure de triméthylsilane et l'ensemble est agité 5 heures à reflux. Après évaporation, le résidu est repris dans 10 ml d'acide chlorhydrique 3N, la suspension est agitée 30 minutes à 80°C et le précipité est filtré. Le produit attendu est obtenu après recristallisation du précipité dans de l'éthanol.
Point de fusion : > 300°C

| Microanalyse élémentaire : | | | | |
|---|---|---|---|---|
| | C % | H % | N % | Cl % |
| calculé | 36,76 | 2,06 | 4,76 | 24,12 |
| trouvé | 36,58 | 2,16 | 4,77 | 24,18 |

### Exemple 2 : Acide 6,7-dichloro-2(1H)-quinoléinone-3-phosphonique

Le produit attendu est obtenu selon le procédé décrit dans l'exemple 1 en utilisant le produit de départ correspondant.
Point de fusion : > 300°C

| Microanalyse élémentaire : | | | | |
|---|---|---|---|---|
| | C % | H % | N % | Cl % |
| calculé | 36,76 | 2,06 | 4,76 | 24,12 |
| trouvé | 36,82 | 2,44 | 4,82 | 24,65 |

### Exemple 3 : Monoéthylester de l'acide 5,7-dichloro-2(1H)-quinoléinone-3-phosphonique

### Stade A : Monoéthylester de l'acide 5,7-dichloro-2-méthoxyquinol-3-yl phosphonique

1,3 mmoles du composé décrit au stade D de l'exemple 1 sont agitées vigoureusement à 100°C dans 5 ml de soude 5N pendant 90 minutes. Après refroidissement, le milieu est acidifié par de l'acide chlorhydrique 1N. Après extraction à l'acétate d'éthyle, lavage de la phase organique par une solution saturée de chlorure de sodium, séchage et évaporation, on obtient une huile incolore qui cristallise lentement et conduit au produit attendu.
Point de fusion : 157-158°C

| Microanalyse élémentaire : | | | | |
|---|---|---|---|---|
| | C % | H % | N % | Cl % |
| calculé | 42,88 | 3,60 | 4,17 | 21,10 |
| trouvé | 42,95 | 3,89 | 4,23 | 21,13 |

### Stade B : Monoéthylester de l'acide 5,7-dichloro-2(1H)-quinoléinone-3-phosphonique

Une suspension contenant 2,2 mmoles du composé décrit au stade précédent dans 16 ml d'acide chlorhydrique 3N est agitée 24 heures à reflux. Le précipité formé est alors filtré et conduit au produit attendu par recristallisation dans de l'isopropanol.
Point de fusion : 220-240°C

| Microanalyse élémentaire : | | | | |
|---|---|---|---|---|
| | C % | H % | N % | Cl % |
| calculé | 41,02 | 3,13 | 4,35 | 22,01 |
| trouvé | 41,34 | 3,16 | 4,47 | 22,14 |

### Exemple 4 : Monoéthylester de l'acide 6,7-dichloro-2(1H)-quinoléinone-3-phosphonique

Le produit attendu est obtenu selon le procédé décrit dans l'exemple 3 en utilisant le produit de départ correspondant.
Point de fusion : 268-276°C

| Microanalyse élémentaire : | | | | |
|---|---|---|---|---|
| | C % | H % | N % | Cl % |
| calculé | 41,02 | 3,13 | 4,35 | 22,01 |
| trouvé | 41,28 | 3,30 | 4,45 | 21,84 |

### Exemple 5 : Acide 5,7-dichloro-2(1H)-quinoléinone-3-méthylphosphinique

### Stade A : Ester éthylique de l'acide 5,7-dichloro-2-méthoxyquinol-3-yl méthylphosphinique

Une solution contenant 1,49 mmoles du composé décrit au stade A de l'exemple 3, 10 µl de diméthylformamide dans 15 ml de dichlorométhane est agitée à 40°C. 3 mmoles de chlorure d'oxalyle diluées dans 1 ml de dichlorométhane sont ajoutées goutte à goutte à cette solution. L'ensemble est maintenu à 40°C une heure puis évaporé sous vide. Le résidu est alors dissous dans 10 ml de tétrahydrofurane anhydre et 580 µl d'une solution 3M de chlorure de méthylmagnésium dans le tétrahydrofurane sont alors ajoutés goutte à goutte. Le milieu réactionnel est agité 90 minutes à température ambiante puis hydrolysé à l'aide de 1 ml d'acide chlorhydrique 1N. L'ensemble est dilué dans 100 ml d'acétate d'éthyle. La phase organique est lavée par de l'acide chlorhydrique 1N puis par de la soude 1N, séchée et évaporée. Le produit attendu est obtenu après purification du résidu par chromatographie sur colonne de silice en utilisant comme éluant un mélange dichlorométhane/éthanol (97/3).

### Stade B : Acide 5,7-dichloro-2(1H)-quinoléinone-3-méthylphosphinique

Le produit attendu est obtenu à partir du composé décrit au stade précédent selon le procédé décrit au stade E de l'exemple 1.
Point de fusion : > 300°C

| Microanalyse élémentaire : | | | | |
|---|---|---|---|---|
| | C % | H % | N % | Cl % |
| calculé | 41,13 | 2,76 | 4,80 | 24,28 |
| trouvé | 40,82 | 2,97 | 5,01 | 24,10 |

### Exemple 6 : Monoisopropylester de l'acide 6,7-dichloro-2(1H)-quinoléinone-3-phosphonique

Le produit attendu est obtenu selon le procédé décrit dans l'exemple 3 en utilisant les produits de départ correspondants.
Point de fusion : 235-238°C

| Microanalyse élémentaire : | | | | |
|---|---|---|---|---|
| | C % | H % | N % | Cl % |
| calculé | 42,88 | 3,60 | 4,17 | 21,10 |
| trouvé | 42,19 | 3,52 | 4,23 | 21,22 |

### Exemple 7 : Monocyclopentylméthylester de l'acide 6,7-dichloro-2(1H)-quinoléinone-3-phosphonique

### Stades A et B :

Les procédés décrits dans ces stades sont identiques à ceux décrits aux stades A et B de l'exemple 1 à partir du produit de départ correspondant.

### Stade C : 3-Bromo-6,7-dichloro-2-éthoxyquinoléine

Le produit attendu est obtenu selon le procédé décrit au stade C de l'exemple 1 mais en remplaçant la solution méthanolique de méthanolate de sodium par une solution éthanolique d'éthanolate de sodium.
Point de fusion : 56-58°C

### Stade D : Monométhylester de l'acide 6,7-dichloro-2-éthoxyquinol-3-yl phosphonique

A une suspension contenant 7,1 mmoles du composé décrit au stade précédent dans 5 ml de tétrahydrofurane anhydre sont ajoutées 14,3 mmoles de triéthylamine, 14,3 mmoles de diméthylphosphite et 0,71 mmoles de tétrakis (triphénylphosphine) palladium. L'ensemble est agité à reflux, sous azote, pendant 12 heures. Le milieu est alors dilué dans 200 ml d'acétate d'éthyle. La phase organique est lavée par de l'acide chlorhydrique 1N puis par une solution saturée de chlorure de sodium, enfin séchée et concentrée à 20 ml. Le produit attendu cristallise lentement.
Point de fusion : 164-166°C

| Microanalyse élémentaire : | | | | |
|---|---|---|---|---|
| | C % | H % | N % | Cl % |
| calculé | 42,88 | 3,60 | 4,17 | 21,10 |
| trouvé | 43,37 | 4,04 | 3,96 | 21,31 |

### Stade E : Monocyclopentylméthylester de l'acide 6,7-dichloro-2(1H)-quinoléinone-3-phosphonique

A une solution contenant 0,28 mmoles du composé décrit au stade précédent dans 2 ml de tétrahydrofurane anhydre sont ajoutées 0,42 mmoles de cyclopentane méthanol et 0,42 mmoles de triphénylphosphine. Après 10 minutes d'agitation, 0,42 mmoles de diisopropylazodicarboxylate sont ajoutées et l'ensemble est agité une heure à température ambiante. 0,7 mmoles de bromure de triméthylsilane sont alors ajoutées goutte à goutte et le mélange est agité à nouveau une heure à température ambiante. Après évaporation, le résidu est repris par 4 ml d'acide chlorhydrique 3N puis agité 12 heures à 100°C. Après refroidissement, 20 ml d'acétate d'éthyle sont ajoutés et le mélange biphasique est agité 10 minutes. La suspension blanche qui se forme est filtrée et conduit au produit attendu.
Point de fusion : 286-288°C

| Microanalyse élémentaire : | | | | |
|---|---|---|---|---|
| | C % | H % | N % | Cl % |
| calculé | 47,89 | 4,29 | 3,72 | 18,85 |
| trouvé | 47,78 | 4,38 | 3,99 | 18,73 |

### Exemple 8 : Acide 6-chloro-7,8,9,10-tétrahydro-2(1H)-benzo[h]quinoléinone-3-phosphonique

Le produit attendu est obtenu selon le procédé décrit dans l'exemple 1.
Point de fusion : > 300°C

| Microanalyse élémentaire : | | | | |
|---|---|---|---|---|
| | C % | H % | N % | Cl % |
| calculé | 49,78 | 4,18 | 4,47 | 11,30 |
| trouvé | 49,78 | 4,31 | 4,48 | 11,46 |

### Exemple 9 : Acide 6-nitro-7,8,9,10-tétrahydro-2(1H)-benzo[h]quinoléinone-3-phosphonique

Le produit attendu est obtenu selon le procédé décrit dans l'exemple 1.
Point de fusion : > 300°C

| Microanalyse élémentaire : | | | |
|---|---|---|---|
| | C % | H % | N % |
| calculé | 48,16 | 4,04 | 8,64 |
| trouvé | 48,06 | 4,14 | 8,48 |

### Exemple 10 : Monoéthylester de l'acide 6-nitro-7,8,9,10-tétrahydro-2(1H)-benzo[h]quinoléinone-3-phosphonique

Le produit attendu est obtenu selon le procédé décrit dans l'exemple 3 à partir du composé obtenu au stade D de l'exemple 9.
Point de fusion : 268-273°C

| Microanalyse élémentaire : | | | |
|---|---|---|---|
| | C % | H % | N % |
| calculé | 51,14 | 4,86 | 7,95 |
| trouvé | 50,92 | 4,71 | 7,60 |

### Exemple 11 : Acide 10-bromo-2(1H)-benzo[g]quinoléinone-3-phosphonique

Le produit attendu est obtenu selon le procédé décrit dans l'exemple 1.
Point de fusion : > 300°C

| Microanalyse élémentaire : | | | | |
|---|---|---|---|---|
| | C % | H % | N % | Cl % |
| calculé | 44,10 | 2,56 | 3,96 | 22,57 |
| trouvé | 44,36 | 2,39 | 4,13 | 21,09 |

### Exemple 12 : Acide 7-nitro-2(1H)-quinoléinone-3-phosphonique

Le produit attendu est obtenu selon le procédé décrit dans l'exemple 1 en utilisant le produit de départ correspondant.
Point de fusion : > 300°C

| Microanalyse élémentaire : | | | |
|---|---|---|---|
| | C % | H % | N % |
| calculé | 40,02 | 2,61 | 10,37 |
| trouvé | 40,51 | 2,47 | 10,07 |

### Exemple 13 : Di(pivaloyloxyméthyl) ester de l'acide 6,7-dichloro-2(1H)quinoléinone-3-phosphonique

A une suspension contenant 2,5 mmoles du composé décrit dans l'exemple 2, on ajoute 7,5 mmoles de triéthylamine et l'ensemble est agité environ 10 minutes à température ambiante jusqu'à dissolution totale. A cette solution, 7,5 mmoles de chlorométhylpivalate sont additionnés goutte à goutte et le milieu réactionnel maintenu une nuit à 70°C. Après retour à température ambiante, 100 ml d'acétate d'éthyle sont ajoutés. Le précipité est filtré, rinçé avec de l'acétate d'éthyle et le filtrat est lavé avec de l'acide chlorhydrique 1N puis une solution saturée de chlorure de sodium etséché. Après évaporation sous vide des solvants, l'huile incolore résiduelle est reprise par 15 ml d'éther éthylique. Le produit attendu cristallise alors lentement.
Point de fusion : 175°C

| Microanalyse élémentaire : | | | | |
|---|---|---|---|---|
| | C % | H % | N % | Cl % |
| calculé | 48,29 | 5,02 | 2,68 | 13,58 |
| trouvé | 48,10 | 4,92 | 2,75 | 13,90 |

### Exemple 14 : Acide 6,7-difluoro-2(1H)-quinoléinone-3-phosphonique

### Stade A : 3-Bromo-6,7-difluoro-2(1H)-quinoléinone

Le produit attendu est obtenu selon le procédé décrit au stade A de l'exemple 1 en remplaçant l'acide 5,7-dichloro-2(1H)-quinoléinone-3-carboxylique par l'acide 6,7-difluoro-2(1H)-quinoléinone-3-carboxylique.
Point de fusion : 265-271°C

### Stade B : Diéthylester de l'acide 6,7-difluoro-2(1H)-quinoléinone-3-phosphonique

A 21,1 mmoles du composé obtenu au stade précédent en suspension dans 100ml de tétrahydrofurane anhydre sont ajoutées 42,2 mmoles de triéthylamine, 42,2 mmoles de diéthylphosphite et 0,25 mmoles de tétrakis (triphénylphosphine)palladium. La réaction est agitée, au reflux sous courant d'azote, jusqu'à évaporation complète du solvant. Le résidu est alors repris dans de l'acétate d'éthyle. Le solide formé est filtré, mis en suspension dans de l'eau et l'ensemble est agité 10 minutes. Le produit attendu est obtenu par filtration et séchage.
Point de fusion : 245-252°C

| Microanalyse élémentaire : | | | |
|---|---|---|---|
| | C % | H % | N % |
| calculé | 49,22 | 4,45 | 4,42 |
| trouvé | 49,07 | 4,10 | 4,56 |

### Stade C : Acide 6,7-difluoro-2(1H)-quinoléinone-3-phosphonique

Le produit attendu est obtenu selon le procédé décrit au stade E de l'exemple 1 à partir du composé obtenu au stade précédent.
Point de fusion : 290-297°C

| Microanalyse élémentaire : | | | |
|---|---|---|---|
| | C % | H % | N % |
| calculé | 41,40 | 2,32 | 5,36 |
| trouvé | 40,77 | 2,27 | 5,26 |

### Exemple 15 : Acide 6,7-diméthyl-2(1H)-quinoléinone-3-phosphonique

### Stade A : 3,8-Dibromo-6,7-diméthyl-2(1H)-quinoléinone

A une suspension contenant 9,2 mmoles d'acide 6,7-diméthyl-2(1H)-quinoléinone-3-carboxylique dans 15 ml de pyridine portée à 70°C, sont additionnées goutte à goutte 55,2 mmoles de brome. Après refroidissement, l'ensemble est versé dans de l'acide chlorhydrique 1N. La phase aqueuse est extraite par de l'acétate d'éthyle. La phase organique est séchée, évaporée et conduit au produit attendu qui est purifié par chromatographie sur colonne de silice en utilisant comme éluant un mélange cyclohexane/acétate d'éthyle (1/1) puis de l'acétate d'éthyle et enfin un mélange acétate d'éthyle/méthanol (98/2).
Point de fusion : 216-223°C

| Microanalyse élémentaire : | | | | |
|---|---|---|---|---|
| | C % | H % | N % | Br % |
| calculé | 39,92 | 2,74 | 4,23 | 48,28 |
| trouvé | 40,35 | 2,72 | 4,24 | 48,21 |

### Stade B : Diéthylester de l'acide 8-bromo-6,7-diméthyl-2(1H)-quinoléinone-3-phosphonique

Le produit attendu est obtenu selon le procédé décrit au stade B de l'exemple 14 à partir du composé décrit au stade précédent.
Point de fusion : 187-195°C

| Microanalyse élémentaire : | | | | |
|---|---|---|---|---|
| | C % | H % | N % | Br % |
| calculé | 46,41 | 4,93 | 3,61 | 20,58 |
| trouvé | 46,54 | 4,87 | 3,61 | 20,84 |

### Stade C : Diéthyl ester de l'acide 6,7-diméthyl-2(1H)-quinoléinone-3-phosphonique

2,5 mmoles du composé obtenu au stade précédent sont hydrogénées à température ambiante, dans 80 ml d'éthanol en présence de 150 mg de Palladium (5 %) sur charbon. Après filtration du catalyseur et évaporation, le résidu est repris dans du dichlorométhane. La phase organique est lavée à l'eau, séchée et évaporée et conduit au produit attendu.
Point de fusion : 165-170°C

| Microanalyse élémentaire : | | | |
|---|---|---|---|
| | C % | H % | N % |
| calculé | 58,25 | 6,52 | 4,53 |
| trouvé | 58,16 | 6,50 | 4,51 |

### Stade D : Acide 6,7-diméthyl-2(1H)-quinoléinone-3-phosphonique

Le produit attendu est obtenu selon le procédé décrit au stade E de l'exemple 1 à partir du produit décrit au stade précédent.
Point de fusion : > 300°C

| Microanalyse élémentaire : | | | |
|---|---|---|---|
| | C % | H % | N % |
| calculé | 52,18 | 4,78 | 5,53 |
| trouvé | 52,05 | 4,81 | 5,60 |

### Etude pharmacologique des dérivés de l'invention

### Activités antineurodégénératives in vivo

### 1/ Ischémie cérébrale globale transitoire chez la Gerbille

L'arrêt complet transitoire de la circulation cérébrale, observé par exemple chez l'homme lors d'infarctus cardiaque, entraîne la mort retardée de neurones dits vulnérables, notamment dans l'hippocampe, zone cérébrale essentielle aux fonctions mnémocognitives. Ce même phénomène est retrouvé expérimentalement lors d'occlusion des 2 artères carotides communes.
Ainsi, chez la Gerbille, l'occlusion transitoire (5 minutes) des 2 carotides entraîne la mort complète des neurones pyramidaux (CA1) de l'hippocampe. Cette mort n'apparaît que 3 à 4 jours après l'ischémie. De nombreux travaux ont montré qu'elle serait due en grande partie à la libération excessive de glutamate lors de la recirculation.
Les effets neuroprotecteurs des composés anti-ischémiques sont objectivés par mesure histologique du nombre de neurones (CA1) ayant résisté à l'ischémie cérabrale.
Le NBQX (30 mg/kg IP) protège 50 % des neurones lorsqu'il est administré 30 minutes avant l'ischémie puis 2 fois par jour jusqu'à la mesure histologique réalisée 4 jours plus tard.
Dans les mêmes conditions, le composé de l'exemple 2 à la dose de 30 mg/kg IP protège totalement de la mort neuronale et protège 50 % des neurones à la dose de 10 mg/kg IP.

### 2/ Ischémie focale définitive chez la Souris

L'occlusion de l'artère sylvienne (ou artère cérébrale moyenne MCA) représente environ 70 à 80 % des accidents vasculaires cérébraux chez l'Homme. Ce type d'ischémie peut être fidèlement reproduit chez la Souris par électrocoagulation de la MCA, ce qui entraîne en 24 heures la formation d'un infarctus cortical parfaitement délimité donc mesurable histologiquement.
A la dose de 30 mg/kg IP, administrée 30 minutes avant et 3 heures après l'ischémie, le NBQX et le composé de l'exemple 2, diminuent de 26 % le volume de l'infarctus.

A cette même dose administrée strictement en traitement curatif, 5 minutes, 1 heure et 2 heures après occlusion, le composé de l'exemple 2 diminue l'infarctus de 23 % alors que le NBQX est sans effet.

### Exemple 17: Composition pharmaceutique

| Formule de préparation pour 1000 comprimés dosés à 10 mg | |
|---|---|
| Composé de l'exemple 2 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 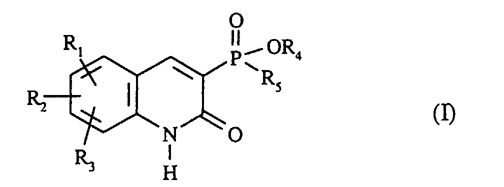 dans laquelle :
R₁, R₂, R₃, identiques ou différents, représentent un atome d'hydrogène, d'halogène, un groupement alkyle (C₁-C₆) linéaire ou ramifié (substitué ou non par un ou plusieurs atomes d'halogène), un groupement nitro, cyano ou aminosulfonyl,
ou bien, lorsque deux d'entre eux sont situés sur des carbones adjacents, forment avec les atomes de carbone auxquels ils sont attachés, un cycle cycloalkyle (C₃-C₇) ou un cycle benzénique (substitué ou non par un ou plusieurs atomes d'halogène, groupements alkyle (C₁-C₆) linéaire ou ramifié, alkoxy (C₁-C₆) linéaire ou ramifié ou trihalogénométhyle),
R₄ représente un atome d'hydrogène, un groupement alkyle (C₁-C₆) linéaire ou ramifié, phényle (substitué ou non par un ou plusieurs atomes d'halogène, groupements alkyle (C₁-C₆) linéaire ou ramifié, alkoxy (C₁-C₆) linéaire ou ramifié ou trihalogénométhyle) ou un groupement 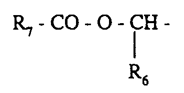 dans lequel R₆ ou R₇, identiques ou différents, représentent un atome d'hydrogène ou un groupement alkyle (C₁-C₆) linéaire ou ramifié (substitué ou non par un groupement cycloalkyle (C₃-C₇) ou phényle),
R₅ représente un atome d'hydrogène, un groupement hydroxy, alkoxy (C₁-C₆) linéaire ou ramifié, phénoxy, mercapto, alkylthio (C₁-C₆) linéaire ou ramifié, alkyle (C₁-C₆) linéaire ou ramifié (substitué ou non par un groupement cycloalkyle (C₃-C₇)), phényle (substitué ou non par un ou plusieurs atomes d'halogène, groupements alkyle (C₁-C₆) linéaire ou ramifié,
alkoxy (C₁-C₆) linéaire ou ramifié ou trihalogénométhyle), amino (substitué ou non par un ou deux groupements alkyle (C₁-C₆) linéaire ou ramifié) ou un groupement 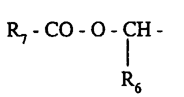 dans lequel R₆ ou R₇ sont tels que définis précédemment,
leurs isomères ainsi que leurs sels d'addition à un acide ou à une base pharmaceutiquement acceptable.

2. Composés de formule (I) selon la revendication 1 tels que l'un au moins des groupements R₁, R₂ ou R₃ représente un atome de chlore.

3. Composés de formule (I) selon la revendication 1 tels que R₄ représente un atome d'hydrogène.

4. Composés de formule (I) selon la revendication 1 tels que R₅ représente un groupement hydroxy.

5. Composé de formule (I) selon la revendication 1 qui est l'acide 6,7-dichloro-2(1H)-quinoléinone-3-phosphonique.

6. Procédé de préparation des composés de formule (I) selon la revendication 1 caractérisé en ce que l'on utilise comme produit de départ un composé de formule (II) : 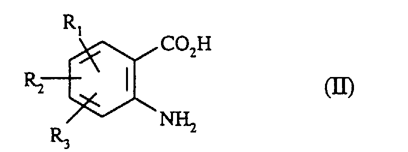 dans laquelle R₁, R₂ et R₃ ont la même signification que dans la formule (I),
que l'on réduit selon la nature des substituants R₁, R₂ et R₃,
soit en milieu aprotique en présence d'hydrure de lithium aluminium, d'hydrure d'aluminium, de diborane ou des complexes de borane,
soit en milieu protique acide lorsque l'on utilise le cyanoborohydrure de sodium, pour conduire à un alcool de formule (III) : 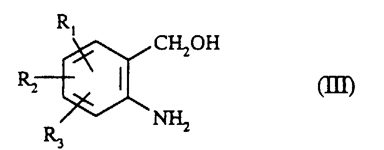 dans laquelle R₁, R₂ et R₃ ont la même signification que dans la formule (I),
que l'on oxyde à l'aide d'un oxyde métallique dans un solvant inerte, d'un hydrohalogénate de métal alcalin dans un solvant protique ou d'un chlorure d'acide dans le diméthylsulfoxyde,
pour conduire à l'aldéhyde de formule (IV) : 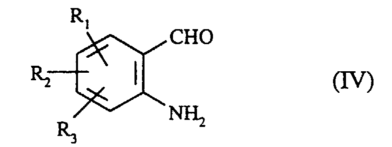 dans laquelle R₁, R₂ et R₃ ont la même signification que dans la formule (I),
qui est alors condensé, avec un malonate d'alkyle de formule (V), en milieu protique, en présence d'un alcoolate de métal alcalin, d'une amine tertiaire ou d'un hydroxyde de métal alcalin : 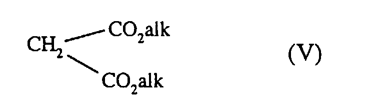 dans laquelle alk représente un groupement alkyle (C₁-C₆) linéaire ou ramifié,
pour conduire à un ester de formule (VI) : 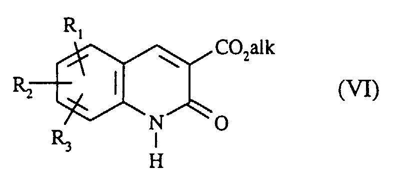 dans laquelle R₁, R₂, R₃ et alk ont la même signification que précédemment,
que l'on transforme, en acide correspondant de formule (VII) : 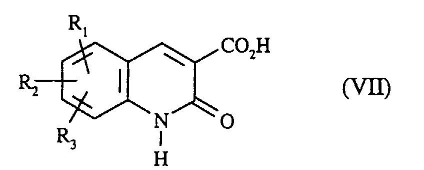 dans laquelle R₁, R₂ et R₃ ont la même signification que dans la formule (I),
que l'on met en réaction en présence de brome dans de la pyridine, pour conduire au composé de formule (VIII) : 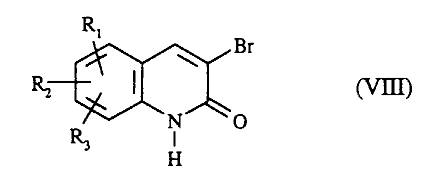 dans laquelle R₁, R₂ et R₃ ont la même signification que dans la formule (I),
dont on protège la fonction lactame par action d'oxychlorure de phosphore suivie de celle d'un alcoolate de métal alcalin en milieu alcoolique, pour conduire au composé de formule (IX) : 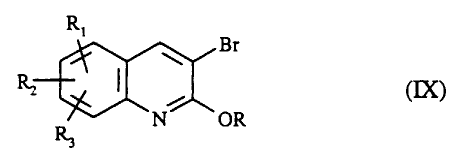 dans laquelle R₁, R₂, R₃ ont la même signification que dans la formule (I) et R représente un groupement alkyle (C₁-C₆) linéaire ou ramifié,
que l'on fait réagir avec un phosphite de dialkyle ou de diaryle, en présence de triéthylamine et de tétrakis(triphényl-phosphine)palladium comme catalyseur, en milieu anhydre, pour conduire au composé de formule (X) : 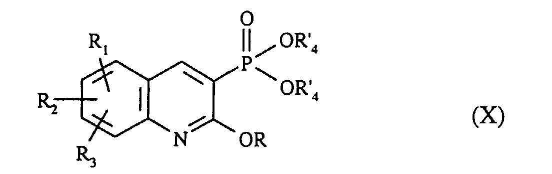 dans laquelle R₁, R₂, R₃ et R ont la même signification que précédemment et R'₄ représente un groupement alkyle (C₁-C₆) linéaire ou ramifié ou un groupement aryle,
qui est ensuite :
soit totalement déprotégé en présence de bromure de triméthylsilane en milieu acétonitrile puis traité en milieu acide,
pour conduire au composé de formule (I/a), cas particulier des composés de formule (I) : 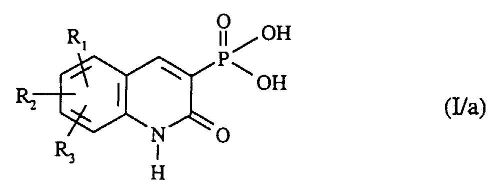 dans laquelle R₁, R₂ et R₃ ont la même signification que dans la formule (I),
que l'on met en réaction si on le souhaite, en milieu basique avec un dérivé halogéné de formule R''₄-Cl dans laquelle R''₄ représente un groupement alkyle (C₁-C₆) linéaire ou ramifié, phényle (substitué ou non par un ou plusieurs atomes d'halogène, groupements alkyle (C₁-C₆) linéaire ou ramifié, alkoxy (C₁-C₆) linéaire ou ramifié ou trihalogénométhyle)
ou un groupement 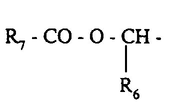 dans lequel R₆ et R₇ ont la même signification que dans la formule (I),
pour conduire au composé de formule (I/b), cas particulier des composés de formule (I), 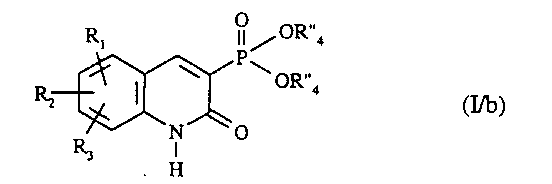 dans laquelle R₁, R₂, R₃ et R''₄ ont la même signification que précédemment,
soit partiellement déprotégé par traitement en milieu basique, pour conduire au composé de formule (XI) : 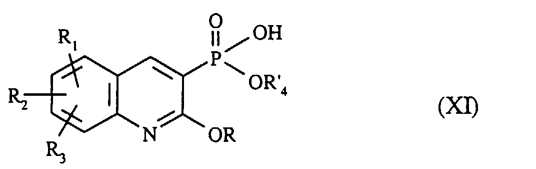 dans laquelle R₁, R₂, R₃, R et R'₄ ont la même signification que précédemment,
composé de formule (XI) :
qui subit, selon la nature des composés de formule (I) que l'on souhaite obtenir :
* ou bien : un traitement en milieu acide, pour conduire au composé de formule (I/c), cas particulier des composés de formule (I) : 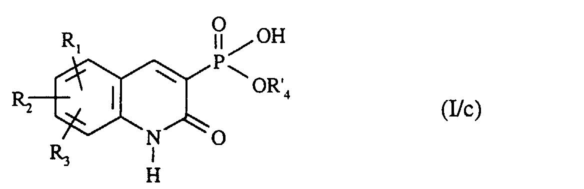 dans laquelle R₁, R₂, R₃ et R'₄ ont la même signification que précédemment,
* ou bien : l'action d'un alcool de formule (XII) :
R'₅-OH (XII)
dans laquelle R'₅ représente un groupement alkyle (C₁-C₆) linéaire ou ramifié ou phényle, pour conduire au composé de formule (XIII) : 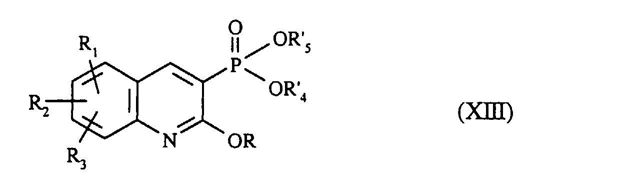 que l'on déprotège sélectivement par action du bromure de triméthylsilane en milieu acétonitrile puis traitement par de l'acide chlorhydrique, pour conduire au composé de formule (I/d), cas particulier des composés de formule (I) : 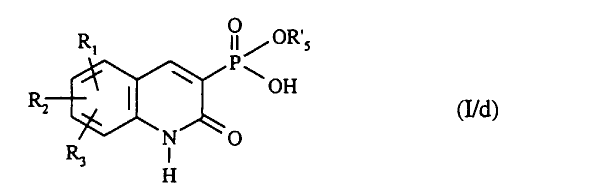 dans laquelle R₁, R₂, R₃ et R'₅ ont la même signification que précédemment,
* ou bien encore : l'action du chlorure d'oxalyle, pour conduire au composé de formule (XIV) : 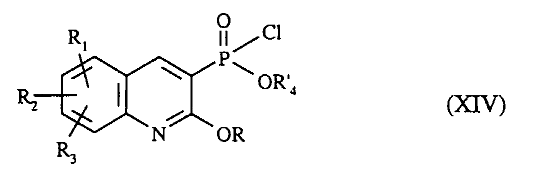 dans laquelle R₁, R₂, R₃, R et R'₄ ont la même signification que précédemment,
composé de formule (XIV) que l'on traite ensuite :
- soit avec un organomagnésien de formule (XV), en milieu anhydre :
R''₅ MgX (XV)
dans laquelle X représente un atome d'halogène et R''₅ représente un groupement alkyle (C₁-C₆) linéaire ou ramifié (substitué ou non par un groupement cycloalkyle (C₃-C₇)) ou phényle (substitué ou non par un ou plusieurs atomes d'halogène, groupements alkyle (C₁-C₆) linéaire ou ramifié, alkoxy (C₁-C₆) linéaire ou ramifié ou trihalogénométhyle), pour conduire au composé de formule (XVI) : 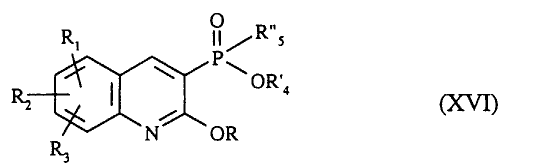 dans laquelle R₁, R₂, R₃, R, R'₄ et R''₅ ont la même signification que précédemment,
que l'on déprotège par action du bromure de triméthylsilane en milieu acétonitrile puis traitement par de l'acide chlorhydrique,
pour conduire au composé de formule (I/e), cas particulier des composés de formule (I) : 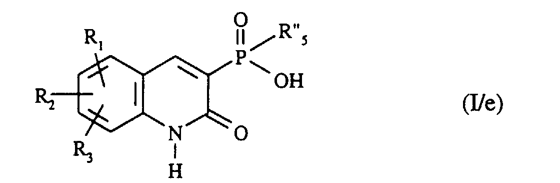 dans laquelle R₁, R₂, R₃ et R''₅ ont la même signification que précédemment,
- soit que l'on met en réaction avec un hydrure, H₂S, alkSH (dans lequel alk signifie un groupement alkyle (C₁-C₆) linéaire ou ramifié) ou de l'ammoniac, pour conduire au composé de formule (XVII) : 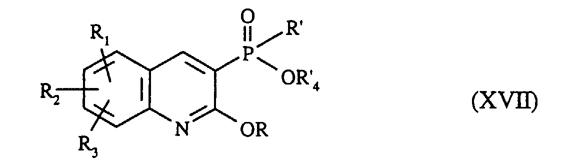 dans laquelle R₁, R₂, R₃, R et R'₄ ont la même signification que précédemment et R' représente HS-, alkS- ou H₂N-, ou un atome d'hydrogène,
que l'on déprotège par action du bromure de triméthylsilane en milieu acétonitrile puis traitement par de l'acide chlorhydrique,
pour conduire au composé de formule (I/f), cas particulier des composés de formule (I) : 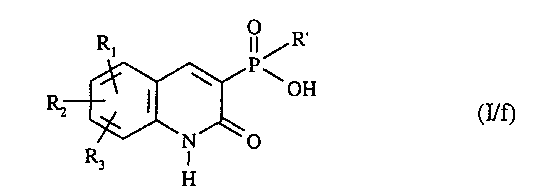 dans laquelle R₁, R₂, R₃ et R' ont la même signification que précédemment,
composé de formule (I/a), (I/b), (I/c), (I/d), (I/e) ou (I/f)
- qui, lorsque R₁ et/ou R₂ et/ou R₃ représentent un atome d'hydrogène, peut subir des substitutions électrophiles, selon des techniques classiques de substitution des noyaux aromatiques conduisant à un composé de formule (I) mono, di ou trisubstitué sur le noyau phényl de la quinoléinone,
- qui, lorsque R₁ et/ou R₂ et/ou R₃ représentent un groupement nitro, peut subir une hydrogénation pour conduire au dérivé amino correspondant, qui, lui-même, peut être, si on le souhaite, transformé en dérivé cyano correspondant,
- qui peut être, le cas échéant, purifié selon une technique classique de purification,
- dont on sépare, le cas échéant, les isomères selon une technique classique de séparation,
- que l'on transforme, si on le souhaite, en ses sels d'addition à un acide ou à une base pharmaceutiquement acceptable.

7. Compositions pharmaceutiques contenant comme principe actif au moins un composé selon l'une quelconque des revendications 1 à 5, seul ou en combinaison avec un ou plusieurs véhicules inertes, non toxiques, pharmaceutiquement acceptables.

8. Compositions pharmaceutiques selon la revendication 7 contenant au moins un principe actif selon l'une des revendications 1 à 5 utiles en tant qu'inhibiteurs des phénomènes pathologiques liés à l'hyperactivation des voies de neurotransmission aux aminoacides excitateurs, dans le traitement de l'accident vasculaire cérabral, du traumatisme cérébral ou spinal, de l'épilepsie et des maladies neurodégénératives chroniques telles que la sclérose amyotrophique latérale, la chorée de Huntington, la schizophrénie et la maladie d'Alzheimer.

## Patentansprüche

1. Verbindungen der Formel (I): 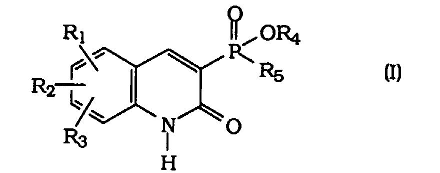 in der:
R₁, R₂ und R₃, die gleichartig oder verschieden sind, jeweils ein Wasserstoffatom, ein Halogenatom, eine geradkettige oder verzweigte (gegebenenfalls durch ein oder mehrere Halogenatome substituierte) (C₁-C₆)-Alkylgruppe. Nitrogruppe, Cyanogruppe oder Aminosulfonylgruppe oder,
wenn zwei dieser Gruppen an benachbarten Kohlenstoffatomen vorliegen, gemeinsam mit den Kohlenstoffatomen, an die sie gebunden sind, einen (C₃-C₇)-Cycloalkylring oder einen (gegebenenfalls durch ein oder mehrere Halogenatome, geradkettige oder verzweigte (C₁-C₆)-Alkylgruppen, geradkettige oder verzweigte (C₁-C₆)-Alkoxygruppen oder Trihalogenmethylgruppen substituierten) Benzolring,
R₄ ein Wasserstoffatom, eine geradkettige oder verzweigte (C₁-C₆)-Alkylgruppe, eine (gegebenenfalls durch ein odermehrere Halogenatome, geradkettige oder verzweigte (C₁-C₆) -Alkylgruppen, geradkettige oder verzweigte (C₁-C₆)-Alkoxygruppen oder Trihalogenmethylgruppen substituierte) Phenylgruppe oder eine Gruppe 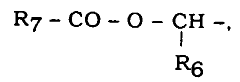 in der R₆ oder R₇, die gleichartig oder verschieden sind, jeweils ein Wasserstoffatom oder eine geradkettige oder verzweigte (gegebenenfalls durch eine (C₃-C₇)-Cycloalkylgruppe oder eine Phenylgruppe substituierte) Alkylgruppe darstellen, und
R₅ ein Wasserstoffatom, eine Hydroxygruppe, eine geradkettige oder verzweigte (C₁-C₆)-Alkoxygruppe, Phenoxygruppe, Mercaptogruppe, geradkettige oder verzweigte (C₁-C₆)-Alkylthiogruppe, geradkettige oder verzweigte (gegebenenfalls durch eine (C₃-C₇)-Cycloalkylgruppe substituierte) Alkylgruppe, eine (gegebenenfalls durch durch ein oder mehrere Halogenatome, geradkettige oder verzweigte (C₁-C₆)-Alkylgruppen, geradkettige oder verzweigte (C₁-C₆)-Alkoxygruppen oder Trihalogenmethylgruppen substituierte) Phenylgruppe, eine (gegebenenfalls durch eine oder zwei geradkettige oder verzweigte (C₁-C₆)-Alkylgruppen substituierte) Aminogruppe oder eine Gruppe 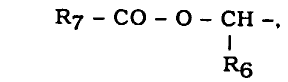 in der R₆ und R₇ die oben angegebenen Bedeutungen besitzen,
bedeuten, deren Isomere sowie deren Additionssalze mit einer pharmazeutisch annehmbaren Säure oder Base.

2. Verbindungen der Formel (I) nach Anspruch 1, in der mindestens eine der Gruppen R₁, R₂ oder R₃ ein Chloratom darstellt.

3. Verbindungen der Formel (I) nach Anspruch 1, worin R₄ ein Wasserstoffatom bedeutet.

4. Verbindungen der Formel (I) nach Anspruch 1, worin R₅ eine Hydroxygruppe bedeutet.

5. Verbindung der Formel (I) nach Anspruch 1, nämlich 6,7-Dichlor-2-(1H)-chinolinon-3-phosphonsäure.

6. Verfahren zur Herstellung der Verbindungen der Formel (I) nach Anspruch 1, dadurch gekennzeichnet, daß man als Ausgangsprodukt eine Verbindung der Formel (II) einsetzt: 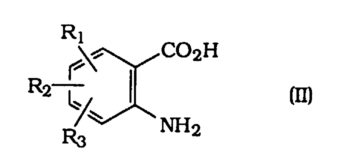 in der R₁, R₂ und R₃ die bezüglich der Formel (I) angegebenen Bedeutungen besitzen,
welche man in Abhängigkeit von der Art der Substituenten R₁, R₂ und R₃ entweder in einem aprotischen Medium in Gegenwart von Lithiumaluminiumhydrid, Aluminiumhydrid, Diboran oder Borankomplexen,
oder in einem sauren protischen Medium, wenn man Natriumcyanoborhydrid verwendet, hydriert zur Bildung eines Alkohols der Formel (III): 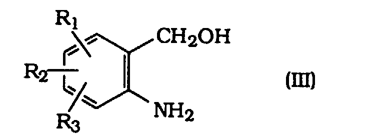 in der R₁, R₂ und R₃ die bezüglich der Formel (I) angegebenen Bedeutungen besitzen,
welchen man mit Hilfe eines Metalloxids in einem inerten Lösungsmittel, einem Alkalimetallhydrohalogenat in einem protischen Lösungsmittel oder einem Säurechlorid in Dimethylsulfoxid oxidiert zur Bildung des Aldehyds der Formel (IV): 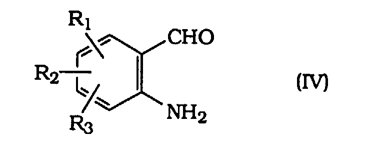 in der R₁, R₂ und R₃ die bezüglich der Formel (I) angegebenen Bedeutungen besitzen,
welchen man anschließend mit einem Malonsäurealkylester der Formel (V): 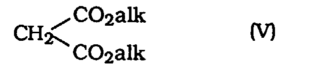 in der alk eine geradkettige oder verzweigte (C₁-C₆)-Alkylgruppe darstellt, in protischem Medium in Gegenwart eines Alkalimetallalkoholats, eines tertiären Amins oder eines Alkalimetallhydroxids kondensiert zur Bildung eines Esters der Formel (VI): 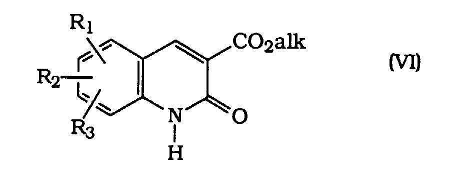 in der R₁, R₂, R₃ und alk die oben angegebenen Bedeutungen besitzen,
welchen man in die entsprechende Säure der Formel (VII) umwandelt: 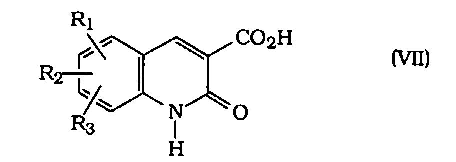 in der R₁, R₂ und R₃ die bezüglich der Formel (I) angegebenen Bedeutungen besitzen,
welche man in Pyridin mit Brom umsetzt zur Bildung der Verbindung der Formel (VIII): 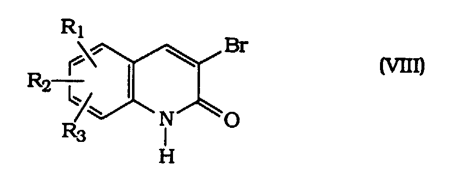 in der R₁, R₂ und R₃ die bezüglich der Formel (I) angegebenen Bedeutungen besitzen,
deren Lactamfunktion man durch Umsetzung mit Phosphoroxidchlorid, gefolgt von einer Umsetzung mit einem Alkalimetallalkoholat in alkoholischem Medium schützt zur Bildung der Verbindung der Formel (IX): 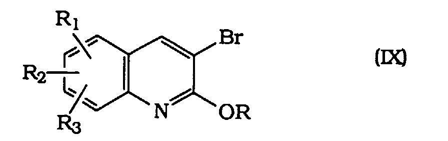 in der R₁, R₂ und R₃ die bezüglich der Formel (I) angegebenen Bedeutungen besitzen und R eine geradkettige oder verzweigte (C₁-C₆)-Alkylgruppe darstellt,
welche man in Gegenwart von Triethylamin und Tetrakis(triphenyl-phosphin)palladium als Katalysator in wasserfreiem Medium mit einem Dialkyl- oder Diarylphosphit umsetzt zur Bildung der Verbindung der Formel (X): 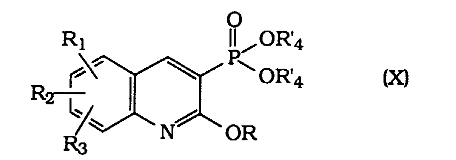 in der R₁, R₂, R₃ und R die oben angegebenen Bedeutungen besitzen und R'₄ eine geradkettige oder verzweigte (C₁-C₆)-Alkylgruppe oder eine Arylgruppe bedeutet, welche man anschließend:
entweder in Gegenwart von Trimethylsilanbromid in einem Acetonitril-Medium und dann durch Behandeln in saurem Medium von den Schutzgruppen befreit,
zur Bildung der Verbindung der Formel (I/a), einem Sonderfall der Verbindungen der Formel (I): 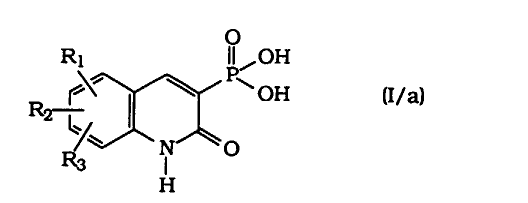 in der R₁, R₂ und R₃ die bezüglich der Formel (I) angegebenen Bedeutungen besitzen,
welche man gewünschtenfalls in basischem Medium mit einem Halogenderivat der Formel R"₄-Cl, in der R"₄ eine geradkettige oder verzweigte (C₁-C₆)-Alkylgruppe, eine (gegebenenfalls durch eines oder mehrere Halogenatome, geradkettige oder verzweigte (C₁-C₆)-Alkylgruppen, geradkettige oder verzweigte (C₁-C₆)-Alkoxygruppen oder Trihalogenmethylgruppen substituierte) Phenylgruppe oder eine Gruppe 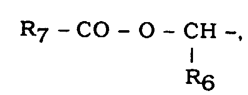 in der R₆ und R₇ die bezüglich der Formmel (I) angegebenen Bedeutungen besitzen, bedeutet, umsetzt zur Bildung der Verbindung der Formel (I/b), einem Sonderfall der Verbindungen der Formel (I): 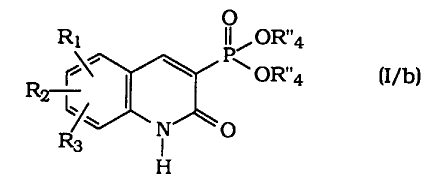 in der R₁, R₂, R₃ und R"₄ die oben angegebenen Bedeutungen besitzen,
oder durch Behandeln in basischem Medium teilweise von den Schutzgruppen befreit zur Bildung der Formel (XI): 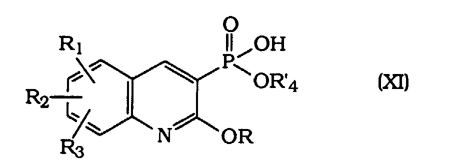 in der R₁, R₂, R₃, R und R'₄ die oben angegebenen Bedeutungen besitzen,
welche Verbindung der Formel (XI) man:
in Abhängigkeit von der Art der zu erhaltenden Verbindungen der Formel (I):
* entweder: einer Behandlung in saurem Medium unterwirft zur Bildung der Verbindung der Formel (I/c), einem Sonderfall der Verbindungen der Formel (I): 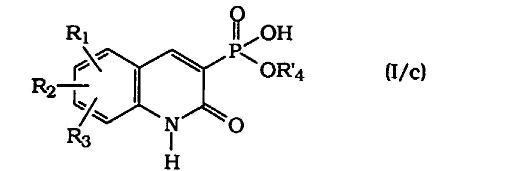 in der R₁, R₂, R₃ und R'₄ die oben angegebenen Bedeutungen besitzen,
* oder: der Einwirkung eines Alkohols der Formel (XII) unterwirft:
R'₅ - OH (XII)
in der R'₅ eine geradkettige oder verzweigte (C₁-C₆)-Alkylgruppe oder eine Phenylgruppe bedeutet, zur Bildung der Verbindung der Formel (XIII): 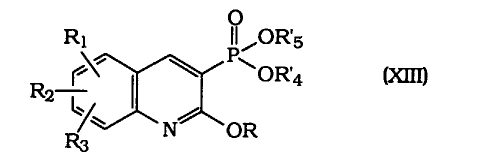 welche man selektiv von ihren Schutzgruppen befreit durch Einwirkung von Trimethylsilanbromid in Acetonitril und dann Behandeln mit Chlorwasserstoffsäure zur Bildung der Verbindung der Formel (I/d), einem Sonderfall der Verbindungen der Formel (I): 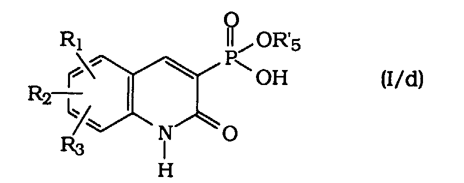 in der R₁, R₂, R₃ und R'₅ die oben angegebenen Bedeutungen besitzen,
* oder schließlich: der Einwirkung von Oxalylchlorid unterwirft zur Bildung der Verbindung der Formel (XIV): 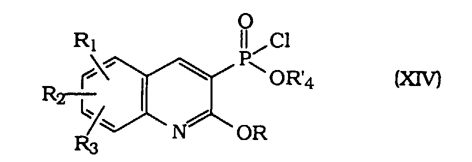 in der R₁, R₂, R₃, R und R'₄ die oben angegebenen Bedeutungen besitzen,
welche Verbindung der Formel (XIV) man anschließend:
- entweder mit einer magnesiumorganischen Verbindung der Formel (XV):
R"₅ MgX (XV)
in der X ein Halogenatom und R"₅ eine geradkettige oder verzweigte (gegebenenfalls durch eine (C₃-C₇)-Cycloalkylgruppe substituierte) Alkylgruppe oder eine (gegebenenfalls durch ein oder mehrere Halogenatome, geradkettige oder verzweigte (C₁-C₆)-Alkylgruppen, geradkettige oder verzweigte (C₁-C₆)-Alkoxygruppen oder Trihalogenmethylgruppen substituierte) Phenylgruppe bedeuten, in wasserfreiem Medium umsetzt zur Bildung der Verbindung der Formel (XVI): 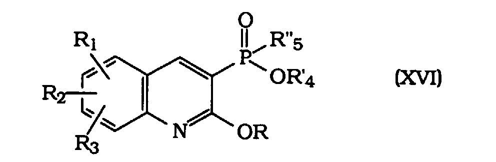 in der R₁, R₂, R₃, R, R'₄ und R"₅ die oben angegebenen Bedeutungen besitzen,
von welcher man durch Einwirkung von Trimethylsilanbromid in Acetonitril als Medium durch Behandeln mit Chlorwasserstoffsäure die Schutzgruppen abspaltet, zur Bildung der Verbindung der Formel (I/e), einem Sonderfall der Verbindungen der Formel (I): 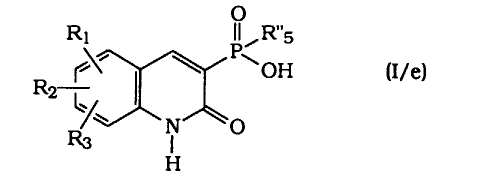 in der R₁, R₂, R₃ und R"₅ die oben angegebenen Bedeutungen besitzen,
- oder welche man mit einem Hydrid, H₂S, alkSH (worin alk eine geradkettige oder verzweigte (C₁-C₆)-Alkylgruppe darstellt) oder Ammoniak umsetzt zur Bildung der Verbindung der Formel (XVII): 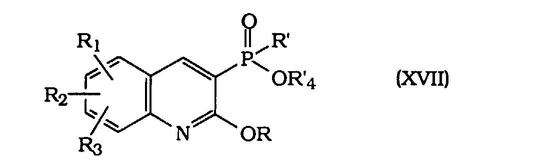 in der R₁, R₂, R₃, R und R'₄ die oben angegebenen Bedeutungen besitzen und R' HS-, alkS- oder H₂N- oder ein Wasserstoffatom bedeutet,
von welcher man durch Einwirkung von Trimethylsilanbromid in Acetonitril als Medium und dann durch Behandeln mit Chlorwasserstoffsäure die Schutzgruppen abspaltet,
zur Bildung der Verbindung der Formel (I/f), einem Sonderfall der Verbindungen der Formel (I): 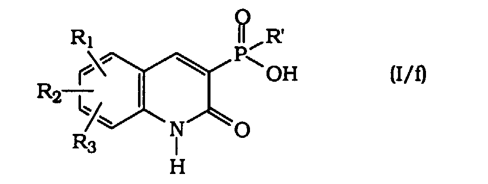 in der R₁, R₂, R₃ und R' die oben angegebenen Bedeutungen besitzen,
welche Verbindungen der Formeln (I/a), (I/b), (I/c), (I/d), (I/e) oder (I/f).
- wenn R₁ und/oder R₂ und/oder R₃ ein Wasserstoffatom bedeuten, man elektrophilen Substitutionen unterwerfen kann unter Anwendung klassischer Methoden zur Substitution aromatischer Kerne zur Bildung der Verbindung der Formel (I), die am Phenylkern des Chinolinons einfach, zweifach oder dreifach substituiert ist,
- wenn R₁ und/oder R₂ und/oder R₃ eine Nitrogruppe bedeuten, man einer Hydrierung unterwerfen kann zur Bildung des entsprechenden Aminoderivats, welches seinerseits gewünschtenfalls in das entsprechende Cyanoderivat umgewandelt werden kann,
- welche man gegebenenfalls mit Hilfe einer klassischen Reinigungsmethode reinigen kann,
- welche man gegebenenfalls mit Hilfe einer klassischen Trennmethode in die Isomeren auftrennen kann und
- welche man gewünschtenfalls mit einer pharmazeutisch annehmbaren Säure oder Base in die Additionssalze überführen kann.

7. Pharmazeutische Zubereitungen enthaltend als Wirkstoffmindestens eine Verbindung nach einem der Ansprüche 1 bis 5 allein oder in Kombination mit einem oder mehreren inerten, nichttoxischen, pharmazeutisch annehmbaren Trägermaterialien.

8. Pharmazeutische Zubereitungen nach Anspruch 7, enthaltend mindestens einen Wirkstoff nach einem der Ansprüche 1 bis 5 als Inhibitoren von pathologischen Zuständen, die mit einer Hyperaktivierung der Neurotransmissionswege für anregende Aminosäuren verknüpft sind, bei der Behandlung von Zerebralgefäßvorfällen, Traumata des Gehirns oder des Rückenmarks, der Epilepsie und von chronischen neurodegenerativen Erkrankungen, wie der amyotrophischen Lateralsklerose, des Huntington-Syndroms, der Schizophrenie und der Alzheimerschen Krankheit.

## Claims

1. Compounds of formula (I) : 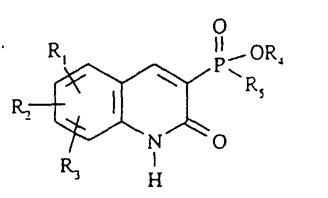 wherein :
R₁ R₂ and R₃, which are identical or different, each represents a hydrogen atom, a halogen atom, a linear or branched (C₁-C₆)-alkyl group (unsubstituted or substituted by one or more halogen atoms), or a nitro, cyano or aminosulphonyl group,
or, when two of those radicals are located at adjacent carbon atoms, those two radicals form with the carbon atoms to which they are attached a (C₃-C₇)-cycloalkyl ring or a benzene ring (unsubstituted or substituted by one or more halogen atoms, linear or branched (C₁-C₆)-alkyl groups, linear or branched (C₁-C₆)-alkoxy groups or trihalomethyl groups),
R₄ represents a hydrogen atom, a linear or branched (C₁-C₆)alkyl group, a phenyl group (unsubstituted or substituted by one or more halogen atoms, linear or branched (C₁-C₆)-alkyl groups, linear or branched (C₁-C₆)-alkoxy groups or trihalomethyl groups) or a grouping 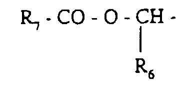 wherein R₆ and R₇, which are identical or different, each represents a hydrogen atom or a linear or branched (C₁-C₆)-alkyl group (unsubstituted or substituted by a (C₃-C₇)-cycloalkyl group or phenyl group),
R₅ represents a hydrogen atom, or one of the following groups: hydroxy, linear or branched (C₁-C₆)-alkoxy, phenoxy, mercapto, linear or branched (C₁-C₆)-alkylthio, linear or branched (C₁-C₆)-alkyl (unsubstituted or substituted by a (C₃-C₇)-cycloalkyl group), phenyl (unsubstituted or substituted by one or more halogen atoms, linear or branched (C₁-C₆)-alkyl groups, linear or branched (C₁-C₆)-alkoxy groups or trihalomethyl groups) or amino (unsubstituted or substituted by one or two linear or branched (C₁-C₆)-alkyl groups) or a grouping 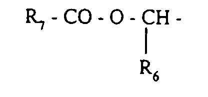 wherein
R₆ and R₇ are as defined above,
their isomers and the additions salts thereof with a pharmaceutically acceptable acid or base.

2. Compounds of formula (I) according to claim 1 wherein at least one of the groups R₁, R₂ and R₃ represents a chlorine atom.

3. Compounds of formula (I) according to claim 1 wherein R₄ represents a hydrogen atom.

4. Compounds of formula (I) according to claim 1 wherein R₅ represents a hydroxy group.

5. Compound of formula (I) according to claim 1 which is 6,7-dichloro-2(1H)-quinolinone-3-phosphonic acid.

6. Process for the preparation of compounds of formula (I) according to claim 1, characterised in that there is used as starting material a compound of formula (II): 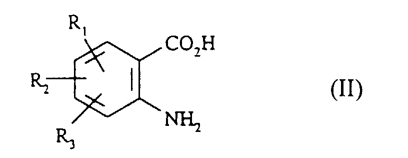 wherein R₁, R₂ and R₃ are as defined for formula (I),
which is reduced, in accordance with the nature of the substituents R₁, R₂ and R₃, either in aprotic medium in the presence of lithium aluminium hydride, aluminium hydride, diborane or complexes of borane,
or in protic acid medium when sodium cyanoborohydride is used,
to yield an alcohol of formula (III) : 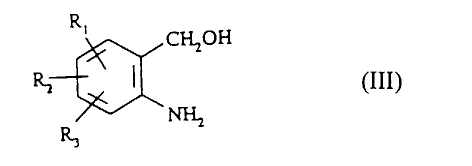 wherein R₁, R₂ and R₃ are as defined for formula (I),
which is oxidised by means of a metal oxide in an inert solvent, a hydrohalogenate of an alkali metal in a protic solvent or an acid chloride in dimethyl sulphoxide,
to yield an aldehyde of formula (IV) : 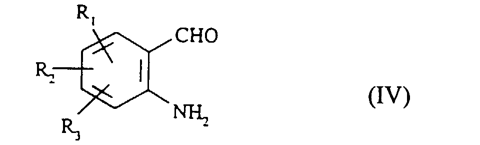 wherein R₁, R₂ and R₃ are as defined for formula (I),
which is then condensed with an alkyl malonate of formula (V): 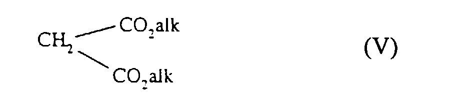 wherein alk represents a linear or branched (C₁-C₆)-alkyl group, in protic medium in the presence of an alkali metal alcoholate, a tertiary amine or an alkali metal hydroxide,
to yield an ester of formula (VI) : 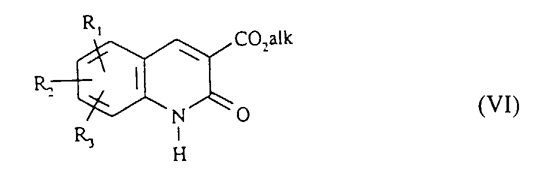 wherein R₁, R₂, R₃ and alk are as defined above,
which is converted into the corresponding acid of formula (VII) : 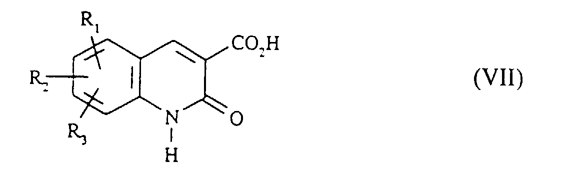 wherein R₁, R₂ and R₃ are as defined for formula (I),
which is reacted in the presence of bromine in pyridine to yield a compound of formula (VIII): 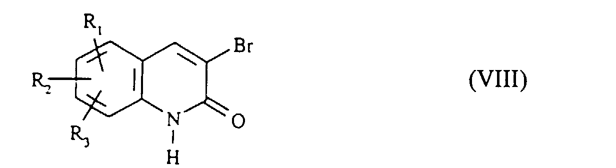 wherein R₁, R₂ and R₃ are as defined for formula (I),
the lactam function of which is protected by the action of phosphorus oxychloride followed by the action of an alkali metal alcoholate in alcoholic medium, to yield a compound of formula (IX) : 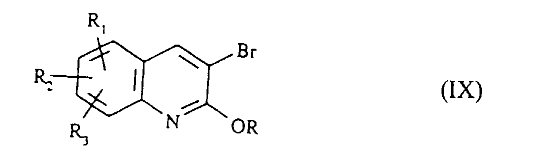 wherein R₁, R₂ and R₃ are as defined for formula (I) and R represents a linear or branched (C₁-C₆)-alkyl group,
which is reacted with a dialkyl or diaryl phosphite in anhydrous medium, in the presence of triethylamine and tetrakis(triphenylphosphine)palladium as catalyst, to yield a compound of formula (X) : 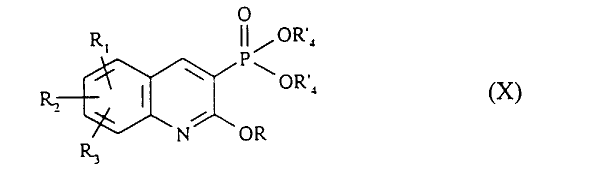 wherein R₁, R₂, R₃ and R are as defined above and R'₄ represents a linear or branched (C₁-C₆)-alkyl group or an aryl group, which is then:
either fully deprotected in the presence of trimethylsilane bromide in acetonitrile medium and then treated in acid medium,
to yield a compound of formula (I/a), a particular case of compounds of formula (I) : 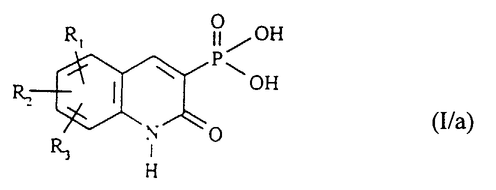 wherein R₁, R₂ and R₃ are as defined for formula (I),
which, if desired, is reacted in basic medium with a halogenated compound of formula R"₄-Cl wherein R"₄ represents a linear or branched (C₁-C₆)-alkyl group, a phenyl group (unsubstituted or substituted by one or more halogen atoms, linear or branched (C₁-C₆)-alkyl groups, linear or branched (C₁-C₆)-alkoxy groups or trihalomethyl groups)
or a grouping 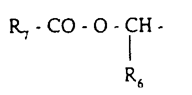 wherein R₆ and R₇ are as defined for formula (I),
to yield a compound of formula (I/b), a particular case of compounds of formula (I) 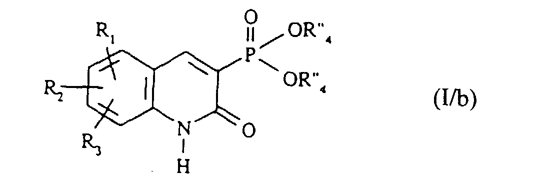 wherein R₁, R₂, R₃ and R"₄ are as defined above,
or partially deprotected by treatment in basic medium to yield a compound of formula (XI) : 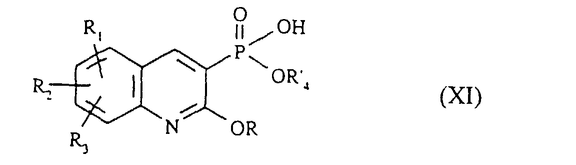 wherein R₁, R₂, R₃, R and R'₄ are as defined above,
which compound of formula (XI):
depending on the nature of the compounds of formula (I) it is desired to obtain is subjected
* to treatment in acid medium to yield a compound of formula (I/c), a particular case of compounds of formula (I): 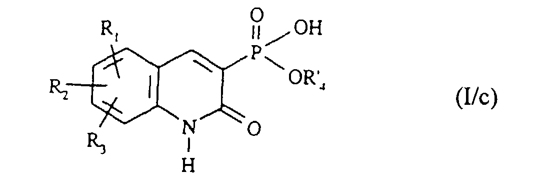 wherein R₁, R₂, R₃ and R'₄ are as defined above,
* to the action of an alcohol of formula (XII):
R'₅-OH (XII)
wherein R'₅ represents a linear or branched (C₁-C₆)-alkyl group or a phenyl group, to yield a compound of formula (XIII) : 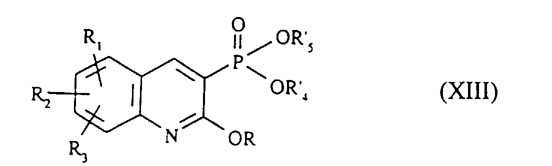 which is selectively deprotected by the action of trimethylsilane bromide in acetonitrile medium followed by treatment with hydrochloric acid, to yield a compound of formula (I/d), a particular case of compounds of formula (I) : 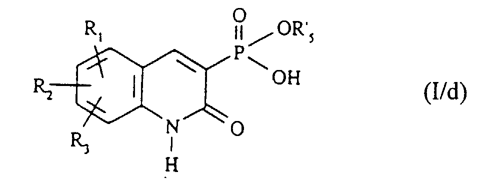 wherein R₁, R₂, R₃ and R'₅ are as defined above,
* or to the action of oxalyl chloride, to yield a compound of formula (XIV) : 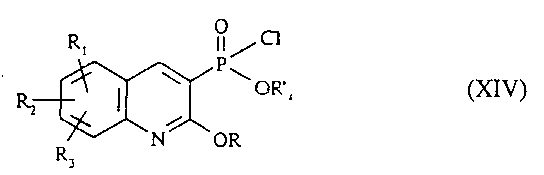 wherein R₁, R₂, R₃, R and R'₄ are as defined above,
which compound of formula (XIV) is then:
- either treated in anhydrous medium with an organomagnesium compound of formula (XV):
R"₅MgX (XV)
wherein X represents a halogen atom and R"₅ represents a linear or branched (C₁-C₆)-alkyl group (unsubstituted or substituted by a (C₃-C₇)-cycloalkyl group) or a phenyl group (unsubstituted or substituted by one or more halogen atoms, linear or branched (C₁-C₆)-alkyl groups, linear or branched (C₁-C₆)-alkoxy groups or trihalomethyl groups) to yield a compound of formula (XVI) : 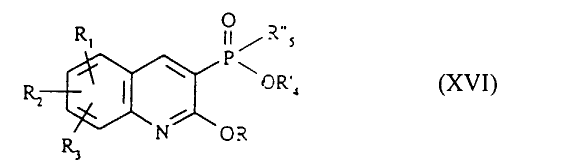 wherein R₁, R₂, R₃, R, R'₄ and R"₅ are as defined above,
which is deprotected by the action of trimethylsilane bromide in acetonitrile medium followed by treatment with hydrochloric acid,
to yield a compound of formula (I/e), a particular case of compounds of formula (I) : 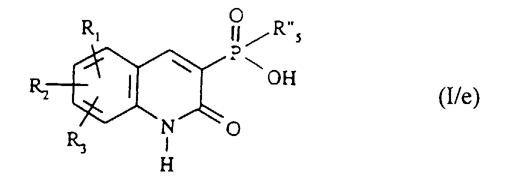 wherein R₁, R₂, R₃ and R"₅ are as defined above,
- or reacted with a hydride, H₂S, alkSH (wherein alk denotes a linear or branched (C₁-C₆)-alkyl group) or ammonia, to yield a compound of formula (XVII) : 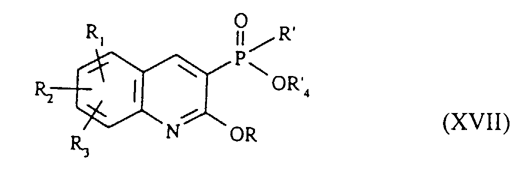 wherein R₁, R₂, R₃, R and R'₄ are as defined above and R' represents HS-, alkS- or H₂N-, or a hydrogen atom,
which is deprotected by the action of trimethylsilane bromide in acetonitrile medium followed by treatment with hydrochloric acid,
to yield a compound of formula (I/f), a particular case of compounds of formula (I): 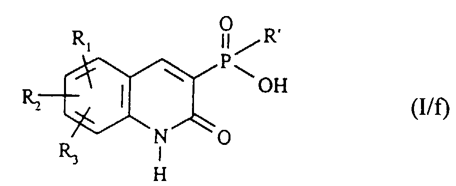 wherein R₁, R₂, R₃ and R' are as defined above,
which compound of formula (I/a), (I/b), (I/c), (I/d), (I/e) or (I/f),
- when R₁ and/or R₂ and/or R₃ represent a hydrogen atom, may be subjected to electrophilic substitutions according to conventional aromatic nuclei substitution techniques yielding a compound of formula (I) mono-, di- or trisubstituted at the phenyl nucleus of the quinolinone,
- when R₁ and/or R₂ and/or R₃ represent a nitro group, may be subjected to hydrogenation to yield the corresponding amino compound which in turn may, if desired, by converted into the corresponding cyano compound,
- may, if necessary, be purified according to a conventional purification technique.
- is separated, if appropriate, into its isomers according to a conventional separation technique,
- is converted, if desired, into its addition salts with a pharmaceutically acceptable acid or base.

7. Pharmaceutical compositions containing as active ingredient at least one compound according to any one of claims 1 to 5, alone or in combination with one or more pharmaceutically acceptable, inert, non-toxic vehicles.

8. Pharmaceutical compositions according to claim 7, containing at least one active ingredient according to any one of claims 1 to 5 for use as inhibitors of pathological phenomena associated with hyperactivation of neurotransmission pathways by excitatory amino acids, in the treatment of cerebral vascular accident, cerebral or spinal traumatism, epilepsy and chronic neurodegenerative disorders, such as amyotrophic lateral sclerosis, Huntington's chorea, schizophrenia and Alzheimer's disease.
